# EUROPEAN PATENT APPLICATION

(11) **EP 2 050 400 A1**
(43) Date of publication of application: **22.04.2009**
(21) Application number: 07791922.3
(22) Date of filing: 03.08.2007
(51) Int. Cl.: A61B 17/10

(54) **SURGICAL STAPLE REMOVER**

(30) Priority: 09.08.2006 JP 2006216832; 15.02.2007 JP 2007035088
(71) Applicant: Max Co., Ltd., Tokyo 103-8502 (JP)
(72) Inventor: HIRANUMA, Toshio, Chuo-ku, Tokyo 103-8502 (JP); HASHIMOTO, Masahiko, Chuo-ku, Tokyo 103-8502 (JP); YAMAGUCHI, Shigenori, Chuo-ku, Tokyo 103-8502 (JP)
(74) Representative: Samson & Partner
(86) International application number: PCT/JP2007/065248
(87) International publication number: WO 2008/018378

(57) **Abstract**

A surgical staple remover includes a staple supporter which is inserted below side portions of a crown of a surgical staple, which is joined to a skin, to support the surgical staple, a pressing slider which presses the crown of the surgical staple, which is supported by the staple supporter, to deform the surgical staple and to remove the surgical staple out from the skin and moves the surgical staple into a storage portion along a staple receiver which is continuously formed from the staple supporter. The number of the surgical staples inside the storage portion can be reliably and easily counted.

## Description

### TECHNICAL FIELD

The present invention relates to a surgical staple remover for removing surgical staples used to suture a skin wound.

### BACKGROUND ART

Generally, when removing surgical staples using a surgical staple remover, the removed staples are required, in view of hygiene management, to be placed in a tray which is set at an appropriate location. However, when transferring the removed surgical staples from a patient's wound to the tray, there is a risk that the surgical staples may drop on the floor and get scattered and lost. Further, because the surgical staples transferred into the tray are exposed, the surgical staples may come in contact with a hand of a nurse or a disposer.

Therefore, in consideration of safety, there has been developed a surgical staple remover which can immediately slide the removed surgical staple to move it into a safe storage portion (see, e.g., JP 2006-158503 A).

Meanwhile, when removing the surgical staples, the removed surgical staples must be counted so that no surgical staples are left unremoved. This is because the number of surgical staples used for suturation is recorded on a medical chart during a surgery, and it can be said that the surgery is completed only after confirming that the same number of surgical staples as the used surgical staples are removed.

However, the number of surgical staples removed cannot be recognized only by storing the surgical staples in the storage portion. Further, even if the storage portion is made transparent, the number of the surgical staples is hard to count when the surgical staples are overlapping each other. Moreover, there has been a problem of miscounting when the number of surgical staples is large.

### DISCLOSURE OF THE INVENTION

One or more embodiments of the present invention provide a surgical staple remover in which the number of surgical staples removed is easily and reliably countable.

According to one or more embodiments of the present invention, a surgical staple remover includes a body having a storage portion, a staple receiver fixed inside the storage portion, a staple supporter formed continuously from the staple receiver to forwardly protrude from the body, and a pressing slider slidably attached to the body. The staple supporter supports lower side portions of a crown of a surgical staple which joined to a skin. The pressing slider presses the crown of the surgical staple, which is supported by the staple supporter, to deform the surgical staple. Further, the pressing slider moves the surgical staple into the storage portion along the staple receiver by rearwardly sliding with respect to the body. The body is formed such that an inner side of the storage portion is visible from outside. The staple receiver is formed with a plurality of engaging indentations, each being enagageable with the surgical staple.

According to one or more embodiments of the present invention, a surgical staple remover includes a body having a storage portion, a staple receiver fixed inside the storage portion, a staple supporter formed continuously from the staple receiver to forwardly protrude from the body, a pressing slider slidably attached to the body, and a counter. The staple supporter supports lower side portions of a crown of a surgical staple which is joined to a skin. The pressing slider presses the crown of the surgical staple, which is supported by the staple supporter, to deform the surgical staple. Further, the pressing slider moves the surgical staple into the storage portion along the staple receiver by rearwardly sliding with respect to the body. The counter counts the surgical staple which is moved into the storage portion by the pressing slider.

### BRIEF DESCRIPTION OF THE DRAWINGS

Fig. 1 is a perspective view of a surgical staple remover according to a first embodiment of the present invention.
Fig. 2 is a longitudinal sectional view of the surgical staple remover shown in Fig. 1.
Fig. 3A is an explanatory view of an illustrative embodiment of removing a surgical staple.
Fig. 3B is another explanatory view of the illustrative embodiment of removing the surgical staple.
Fig. 4 is a longitudinal sectional view of the surgical staple remover taking in the removed surgical staples.
Fig. 5 is a partially enlarged view of Fig. 4.
Fig. 6 is a perspective view of a surgical staple remover according to a second embodiment of the present invention.
Fig. 7 is a longitudinal sectional view of the surgical staple remover shown in Fig. 6 taking in a surgical staple.
Fig. 8 is a partially enlarged view of Fig. 7.

### EXPLANATION OF REFERENCE NUMERALS

- 1: surgical staple remover
- 3: pressing slider
- 4: supporting member
- 6: storage portion
- 17: staple supporter
- 18: staple receiver
- 20: surgical staple
- 22: engaging indentation
- 23: counter

### BEST MODE FOR CARRYING OUT THE INVENTION

Hereinafter, embodiments of the present invention will be described with reference to the drawings.

### FIRST EMBODIMENT

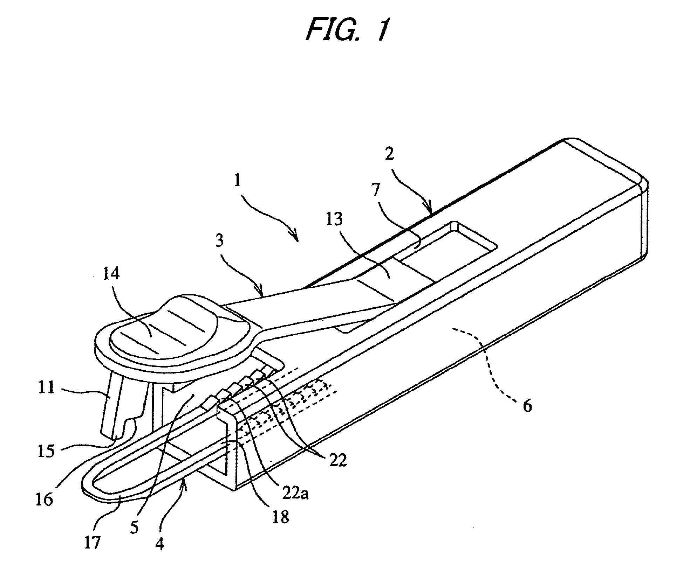

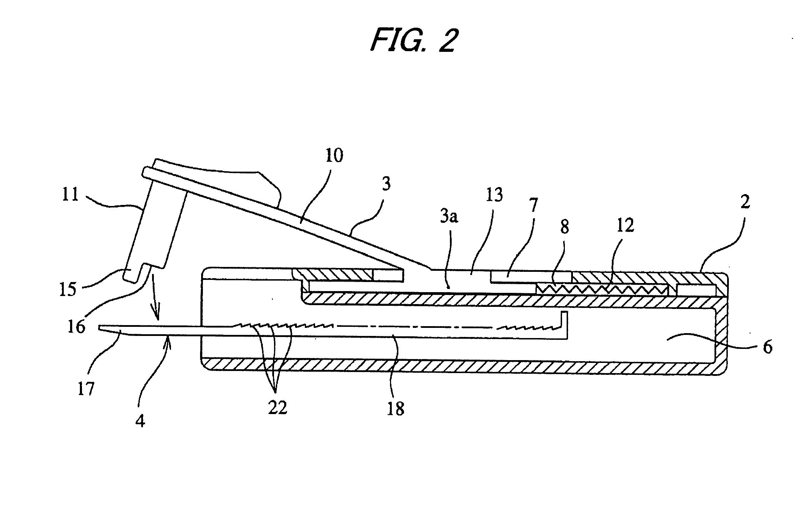

Fig. 1 is a perspective view of a surgical staple remover according to a first embodiment of the present invention, and Fig. 2 is a longitudinal sectional view thereof. As shown in Fig. 1, the surgical staple remover 1 includes a body 2, a pressing slider 3 and a supporting member 4.

The body 2 is formed of transparent synthetic resin in a slender box shape. At the front end of the body 2, a take-in opening 5 is formed. Further, the body 2 has a storage portion 6 inside which a space is formed. The take-in opening 5 is communicated with the storage portion 6. At the central portion of an upper part of the body 2, a guide groove 7 is formed along the longitudinal direction of the body 2. Below the guide groove 7, a guiding passage 8 is formed. The body 2 may not necessarily be transparent in so far as the inner side of the storage portion 6 is visible from outside. For example, the storage portion 6 may be formed with a slit-shaped see-through window through which the inner side can be seen.

The pressing slider 3 includes a slider block 3a, a pressing lever 10 and a staple pusher 11. The slider block 3a is slidably provided in the guiding passage 8 and is constantly biased forward by a spring 12. On top of the slider block 3a, a protrusion 13 is formed. The protrusion 13 is engaged with the guide groove 7 and limits the moving end of the slider block 3a when sliding from the front side toward the rear side.

The pressing lever 10 protrudes obliquely forward from the front end of the protrusion 13 and is formed with a finger rest 14 at a front distal portion thereof. The pressing lever 10 is made of an elastic material so that it is deformable by a downward deflection from a base portion thereof.

The staple pusher 11 protrudes downward below the finger rest 14. A distal head portion of the staple pusher 11 is formed with an engaging part 15, and a pressing part 16 is formed to a rear side of the engaging part 15. The engaging part 15 protrudes more downward than the pressing part 16. The staple pusher 11 is preferably formed of a hard material such as metal because it is required to have strength. However, the staple pusher 11 may be formed of a resin material as long as it has sufficient strength and rigidity to press down a crown 20a of a surgical staple 20.

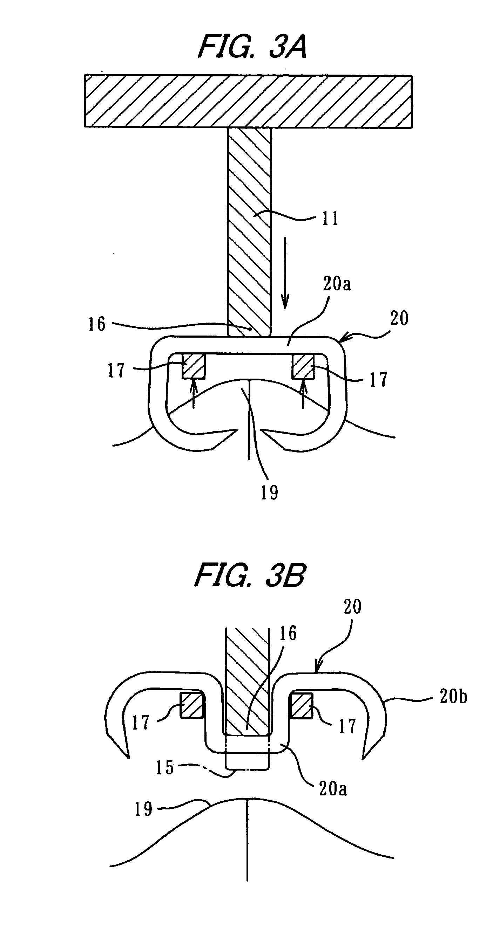

The supporting member 4 is made of metal and has a U-shaped staple supporter 17 at a front portion thereof. The supporting member 4 further has a pair of parallel staple receivers 18 which are continuously formed from the staple supporter 17 in a rearward direction. As shown in Fig. 3A, the staple supporter 17 is inserted between a surgical staple 20, which has been bent by a surgical stapler, and a skin 19 to support both sides of the crown 20a of the surgical staple 20 from below. The width of the staple supporter 17 is slightly narrower than the width of the crown 20a of the surgical staple 20. Further, the staple supporter 17 is formed to have a thin wall thickness so that it can be easily inserted between the crown 20a and the skin 19.

The staple supporter 17 protrudes forward from the take-in opening 5. The staple receivers 18 are arranged inside the storage portion 6 and are secured by suitable means. The staple receivers 18 are formed with continuous engaging indentations 22. The engaging indentations 22 are formed between serrated teeth, and the interval between the engaging indentations 22 that are adjacent to each other in the front-rear direction is set to have a length about 1 to 1.5 times as wide as the thickness of the staple 20.

The foremost (nearest to the staple supporter 17) engaging indentations 22a are set to be positioned substantially immediately above the rear face of the engaging part 15 when the pressing slider 3 is slid to the rear side moving end. The number of the engaging indentations 22 may be determined in accordance with the number of surgery staples used in a usual surgical operation (about 30 to 60).

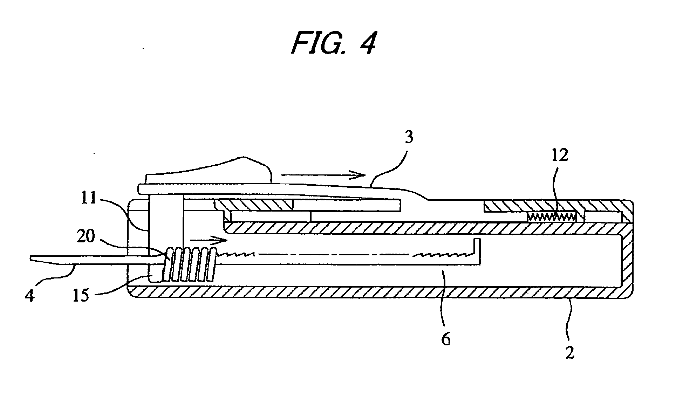

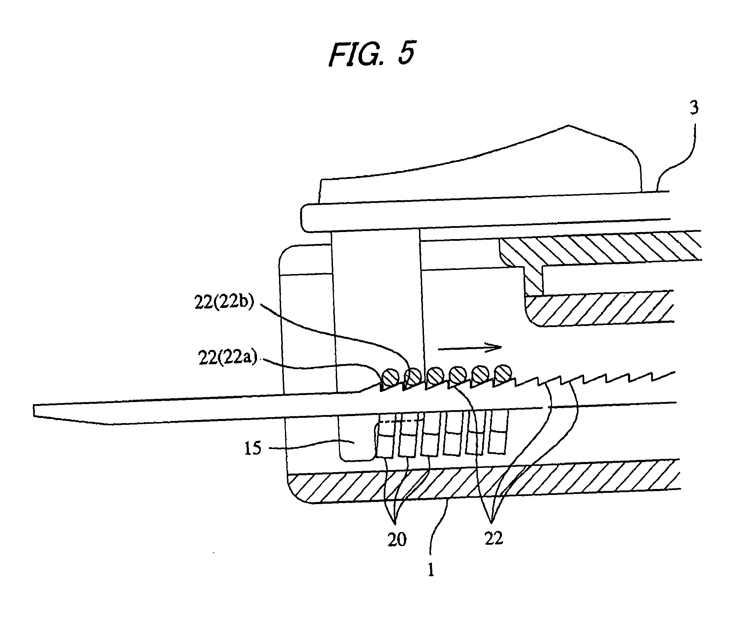

Next, the operation of the surgical staple remover 1 will be described. First, holding the body 2 with one hand, the staple supporter 17 of the supporting member 4 is inserted below the crown 20a of the surgical staple 20 used to suture the wound as shown in Fig. 3A to support the lower portions of both sides of the crown 20a with the staple supporter 17. Next, placing the finger tip on the finger rest 14 of the pressing slider 3, the pressing lever 10 is deflected downward, whereby the pressing part 16 of the staple pusher 11 engages with the upper central portion of the crown 20a of the surgical staple 20. When the pressing lever 10 further deflected downward by applying a force onto the finger rest 14, the upper central portion is pressed downward by the pressing part 16 from above while the lower side portions being supported by the staple supporter 17, whereby the crown 20a of the surgical staple 20 is deformed to be bent in a U-shape as shown in Fig. 3B. As a result, the entire surgical staple 20 is deformed in an M-shape, and the legs 20b on respective sides are removed from the skin 19. After removing the surgical staple 20 in this way, the pressing slider 3 is rearwardly slid against the spring 12 as shown in Fig. 4, whereby the surgical staple 20 engages with the engaging part 15 of the staple pusher 11 and is taken into the storage portion 6 from the take-in opening 5. When the pressing slider 3 is slid to the moving end, the rear face of the engaging part 15 of the pressing slider 3 is located at the position corresponding to the foremost engaging indentations 22a as shown in Fig. 5. Accordingly, the surgical staple 20 thus taken in is supported in engagement with the foremost engaging indentations 22a. Thereafter, when the force is released from the pressing slider 3, the pressing slider 3 is moved to be restored to its initial position by the elastic force of the spring 12. Even when the pressing slider 3 is returned to the initial position, the surgical staple 20 is still stably supported by the pair of parallel staple receivers 18 and is engaged with the engaging indentations 22 each formed between the serrated teeth. Thus, the surgical staple 20 cannot move in a returning direction (in a forward direction) and is held in the same position.

Similarly, the subsequent surgical staple 20 is removed by the operation of the pressing slider 3, and is taken into the storage portion 6 from the take-in opening 5 by the rearward slide of the pressing slider 3. Further, when the pressing slider 3 reaches the moving end, the surgical staple 20 taken in is pushed into the foremost engaging indentations 22a. At this time, the preceding surgical staple 20 which has been held in the engaging indentations 22a is forcibly pushed out rearward. Since the rear sides of the engaging indentations 22 are gently sloped, the surgical staple 20 pushed out is smoothly pushed into engaging indentations 22b on the rear side. Because the interval between the adjacent engaging indentations 22 is set to have a length of about 1 to 1.5 times as wide as the thickness of the staple 20, the pushed out surgical staple 20 is supported in engagement with the subsequent engaging indentation 22b. Thereafter, when a force is released from the pressing slider 3 to restore the pressing slider 3 back to the initial position by the elastic force of the spring 12, the surgical staples 20 engaged in the two sets of engaging indentations 22a, 22b are held in their respective positions. Likewise, the removed surgical staples 20 are held in such a manner that they are sequentially arranged in the engaging indentations 22 of the staple receivers 18 toward the rear side.

When the final surgical staple 20 is engaged in the foremost engaging indentations 22a, all of the removed surgical staples 20 are held in the engaging indentations 22 of the pair of parallel staple receivers 18, so that they are stably held in their orderly arranged state. Therefore, the number of the surgical staples 20 removed can be counted by visually checking from the side of the body 2. In this way, a surgeon can easily and reliably confirm whether or not the number of the surgical staples used for the suturation is equal to the number of the surgical staples removed.

The height of the storage portion 6 is preferably slightly higher than the height of the removed surgical staples 20. More specifically, the storage portion 6 is preferably formed such that the upper face of the storage portion 6 is slightly higher than the upper edges of the surgical staples 20 supported on the staple receivers 18 and such that the lower face of the storage portion 6 is slightly lower than the lower ends of the surgical staple 20 supported on the staple receivers 18. According to such a configuration, even when the surgical staple remover 1 is slightly tilted or moved, the surgical staples 20 held inside the storage portion 6 are unlikely to move out of the respective engaging indentations 22 and are firmly held and supported on the staple receivers 18.

The engaging indentations 22 may not necessarily be serrated. For example, the engaging indentations 22 may be in a continuously corrugated form.

Further, for each length corresponding to a certain number (e.g. 10) of the surgical staples 20 supported side by side on the staple receivers 18, the body 2 or the staple receivers 18 may be changed in color or calibrated so that the surgical staples 20 can be counted quickly and reliably.

### SECOND EMBODIMENT

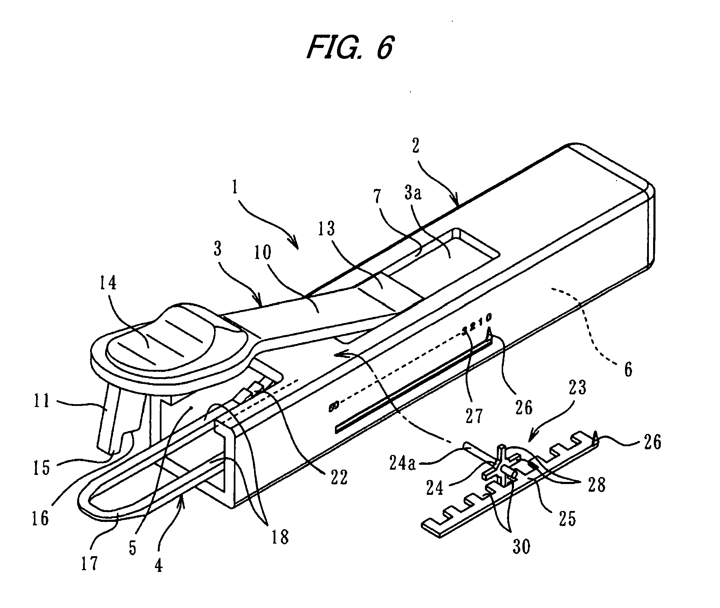

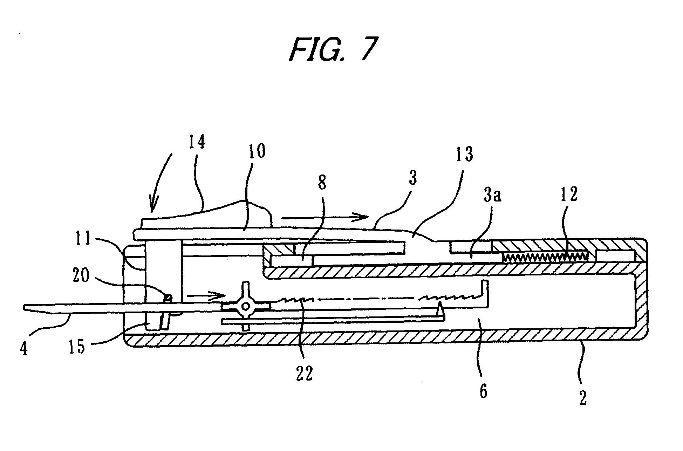

Fig. 6 is a perspective view of a surgical staple remover according to a second embodiment of the present invention, and Fig. 7 is a longitudinal sectional view thereof. In the second embodiment, components that are similar to those of the first embodiment are denoted by same reference numerals, and explanation thereof will be omitted.

The surgical staple remover 1 according to the second embodiment further includes a counter 23 which counts the surgical staples 20 that are rearwardly moved from the staple supporter 17 of the supporting member 4 by the pressing slider 3.

As shown in Fig. 6, the counter 23 includes a gear wheel 24 which is enagageable with the surgical staples 20 that are rearwardly moving from the staple supporter 17 by the pressing slider 3, a rack member 25 interlocks with the rotation of the gear wheel 24, an indicator 26 provided on an end portion of the rack member 25, and a scale 27 marked on one of the sidewalls of the storage portion 6. The gear wheel 24 has four teeth 28 formed in the shape of a cross, one of which meshing with one of indent grooves 30 formed on the rack member 25. A rotating axis 24a of the gear wheel 24 is rotatably supported inside the storage portion 6 by suitable means. When the gear wheel 24 rotates, the rack member 25 and indicator 26 also move interlockingly therewith. The scale 27 is provided along a direction in which the indicator 26 moves along the inner face of the side wall of the storage portion 6. Further, at least a portion of the side wall of the storage portion 6 around the scale 27 is formed such that the indicator 26 is visible.

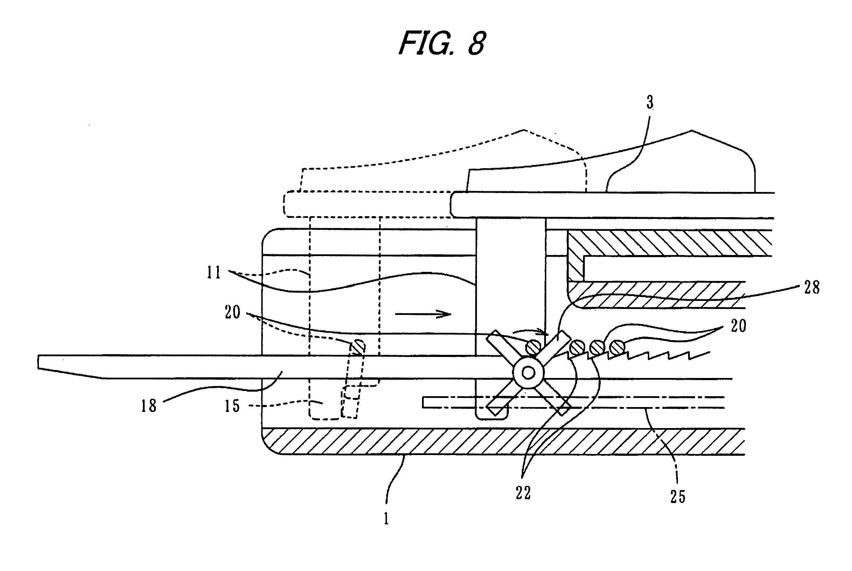

As described above, the surgical staples 20 moving rearward from the staple supporter 17 of the supporting member 4 by the pressing slider 3 are counted by the counter 23. Specifically, as shown in Figs. 7 and 8, when the surgical staple 20 moves along the staple receivers 18, it engages with one of the teeth 28 of the gear wheel 24, whereby the gear wheel 24 is rotated. When the gear wheel 24 rotates, as shown in Figs. 6 and 8, the tooth 28, which is located on the opposite side of the tooth 28 engaging with the surgical staple 20, forwardly pushes the rack member 25, whereby the rack member 25 and the indicator 26 are moved forward along the scale 27 by one scale unit. In this way, when the gear wheel 24 rotates, the indicator 26 interlockingly moves by one unit of the scale 27 so that one surgical staple 20 is counted. When the subsequent surgical staple 20 is taken in, the number of the surgical staples 20 is similarly counted up by one, and the number of the surgical staples 20 is indicated by the indicator 26 and the scale 27. Accordingly, the total number of the removed surgical staples 20 can be easily determined by the indication of the indicator 26 and the scale 27.

According to the second embodiment, the number of the surgical staples 20 removed is automatically counted and indicated. Therefore, it is not necessary to directly look and count the surgical staples one by one. Thus, a surgeon can easily and reliably confirm whether or not the number of the surgical staples 20 used for the suturation is equal to the number of the surgical staples 20 removed.

The counter 23 includes the gear wheel 24 enagageable with the surgical staple 20 which is rearwardly moved by the pressing slider 3, and the indicator 26 which moves interlockingly with the rotation of the gear wheel 24. That is, since the number of the surgical staples 20 removed is mechanically counted and indicated, no electric power supply is required.

The configuration of the counter 23 is not limited to the configuration described above. For example, the counter may be configured in an arc shape. Further, electrical contacts may be provided at positions through which the surgical staple 20 passes, and the counter may count up the number of the surgical staples 20 when the surgical staple 20 taken in is brought into contact with the electrical contacts so that a current is carried therethrough. In this case, because the mechanical portions can be reduced, an increase in the weight of the surgical staple remover can be minimized.

While the present invention has been described in detail with reference to specific embodiments, it is apparent to those skilled in the art that various changes and modifications can be made therein without departing from the spirit and scope of the present invention.

This application is based on Japanese Patent Application No. 2006-216832 filed on August 9, 2006 and Japanese Patent Application No. 2007-035088 filed on February 15, 2007, the contents of which are incorporated herein by reference.

### INDUSTRIAL APPLICABILITY

A surgical staple remover in which the number of surgical staples removed is reliably and safely countable can be provided.

## Claims

1. A surgical staple remover comprising:
a body comprising a storage portion;
a staple receiver fixed inside the storage portion;
a staple supporter formed continuously from the staple receiver to forwardly protrude from the body; and
a pressing slider slidably attached to the body,
wherein the staple supporter is configured to support lower side portions of a crown of a surgical staple which is joined to a skin,
the pressing slider is configured to press the crown of the surgical staple, which is supported by the staple supporter, to deform the surgical staple, and to move the surgical staple into the storage portion along the staple receiver by rearwardly sliding with respect to the body,
the body is formed such that an inner side of the storage portion is visible from outside, and
the staple receiver is formed with a plurality of engaging indentations, each being enagageable with the surgical staple.

2. The surgical staple remover according to claim 1, wherein the engaging indentation nearest to the staple supporter is formed at a position where the surgical staple, which is moved by the pressing slider, is engageable when the pressing slider is slid to the rearmost position.

3. The surgical staple remover according to claim 1, wherein the plurality of engaging indentations are serrated.

4. The surgical staple remover according to claim 1, wherein the body is formed of a transparent material.

5. The surgical staple remover according to claim 1, further comprising a counter configured to count the surgical staple which is moved into the storage portion by the pressing slider.

6. The surgical staple remover according to claim 5, wherein the counter comprises:
a gear wheel rotatably supported inside the storage portion;
an indicator which moves interlockingly with a rotation of the gear wheel; and
a scale provided along a direction in which the indicator moves,
wherein the gear wheel rotates when the surgical staple engages with the gear wheel while the surgical staple is being moved into the storage portion by the pressing slider.

7. The surgical staple remover according to claim 5, wherein the counter counts up the surgical staple when a current is carried through the surgical staple while the surgical staple is being moved into the storage portion by the pressing slider.

8. A surgical staple remover comprising:
a body comprising a storage portion;
a staple receiver fixed inside the storage portion;
a staple supporter formed continuously from the staple receiver to forwardly protrude from the body;
a pressing slider slidably attached to the body; and
a counter,
wherein the staple supporter is configured to support lower side portions of a crown of a surgical staple which is joined to a skin,
the pressing slider is configured to press the crown of the surgical staple, which is supported by the staple supporter, to deform the surgical staple, and to move the surgical staple into the storage portion along the staple receiver by rearwardly sliding with respect to the body, and
the counter counts the surgical staple which is moved into the storage portion by the pressing slider.

9. The surgical staple remover according to claim 8, wherein the counter comprises:
a gear wheel rotatably supported inside the storage portion;
an indicator which-moves interlockingly with a rotation of the gear wheel; and
a scale provided along a direction in which the indicator moves,
wherein the gear wheel rotates when the surgical staple engages with the gear wheel while the surgical staple is being moved into the storage portion by the pressing slider.

10. The surgical staple remover according to claim 8, wherein the counter counts up the surgical staple when a current is carried through the surgical staple while the surgical staple is being moved into the storage portion by the pressing slider.
